(19) Europäisches Patentamt

European Patent Office

Office européen des brevets

(11) Publication number: **0 613 904 A2**

# (12) EUROPEAN PATENT APPLICATION

(21) Application number: **94100793.2**

(22) Date of filing: **20.01.94**

(51) Int. Cl.5: **C07K 7/10, A61K 37/02**

(30) Priority: **22.01.93 JP 9468/93**

(43) Date of publication of application:
**07.09.94 Bulletin 94/36**

(84) Designated Contracting States:
**AT BE CH DE DK ES FR GB IE IT LI LU NL PT SE**

(71) Applicant: **SANWA KAGAKU KENKYUSHO CO., LTD.**
**No. 35, Higashi-sotobori-cho**
**Higashi-ku Nagoya-shi Aichi-ken (JP)**

(72) Inventor: **Takahashi, Haruo, c/o Sanwa Kagaku Kenkyusho**
**Co., Ltd.,**
**35, Higashisotobori-cho,**
**Higashi-ku**
**Nagoya-shi, Aichi (JP)**
Inventor: **Kobayashi, Yohei, c/o Sanwa Kagaku Kenkyusho**
**Co., Ltd.,**
**35, Higashisotobori-cho,**
**Higashi-ku**
**Nagoya-shi, Aichi (JP)**
Inventor: **Nakoshi, Masanao, c/o Sanwa Kagaku Kenkyusho**
**Co., Ltd.,**

**35, Higashisotobori-cho,**
**Higashi-ku**
**Nagoya-shi, Aichi (JP)**
Inventor: **Mitani, Takahiko, c/o Sanwa Kagaku Kenkyusho**
**Co., Ltd.,**
**35, Higashisotobori-cho,**
**Higashi-ku**
**Nagoya-shi, Aichi (JP)**
Inventor: **Hirade, Kinya, c/o Sanwa Kagaku Kenkyusho**
**Co., Ltd.,**
**35, Higashisotobori-cho,**
**Higashi-ku**
**Nagoya-shi, Aichi (JP)**
Inventor: **Sawai, Kiichi, c/o Sanwa Kagaku Kenkyusho**
**Co., Ltd.,**
**35, Higashisotobori-cho,**
**Higashi-ku**
**Nagoya-shi, Aichi (JP)**

(74) Representative: **Weisert, Annekäte, Dipl.-Ing. Dr.-Ing. et al**
**Patentanwälte**
**Kraus Weisert & Partner**
**Thomas-Wimmer-Ring 15**
**D-80539 München (DE)**

(54) **Vasoactive intestinal peptide analogs for treating smooth muscle disorders and/or asthma.**

㉗ A polypeptide represented by formula (I):

$$\text{His-Ser-Asp-Ala-Val-Phe-Thr-R}^1\text{-R}^2\text{-Tyr-Thr-R}^3\text{-Leu-Arg-}$$

$$\text{Lys-Gln-Leu-Ala-R}^4\text{-R}^5\text{-Lys-Tyr-Leu-R}^6\text{-R}^7\text{-R}^8\text{-R}^9\text{-R}^{10}\text{-X} \quad (I)$$

wherein $R^1$ represents Asp or Gly; $R^2$ represents Asn or Gln; $R^3$ represents Arg or Lys; $R^4$ represents Val, Ala or Lys; $R^5$ represents Lys or Leu; $R^6$ represents Asn, Gln or Lys; $R^7$ represents Ser, Lys or Ala; $R^8$ represents Ile, Ala or Leu; $R^9$ represents Leu or Lys; $R^{10}$ represents Asn, Lys or Arg; and X represents homoserine or homoserine lactone, which exhibits higher biological activities and higher stability than conventional VIP analogues.

EP 0 613 904 A2

FIELD OF THE INVENTION

This invention relates to a polypeptide having potent smooth muscle relaxing activity with excellent stability and long duration and to use of the same. The polypeptide of the present invention is useful as an active ingredient of drugs, particularly for prevention and treatment of asthma.

BACKGROUND OF THE INVENTION

Among a number of polypeptides having smooth muscle relaxing activity which have been so far discovered, vasoactive intestinal polypeptide (hereinafter abbreviated as VIP) is a peptide hormone composed of 28 amino acid residues which was isolated from a side fraction in the course of extraction and purification of secretin from the porcine duodenum in 1970 (see S.I. Said and V. Mutt, Eur. J. Biochem., vol. 28, pp. 199-204 (1972)). The amino acid sequence of VIP was elucidated in 1974 (see V. Mutt and S.I. Said, Eur. J. Biochem., vol. 42, pp. 581-589 (1974)), and because of its structural similarity to secretin, glucagon, etc. VIP has been recognized as a peptide hormone belonging to the glucagon-secretin family.

VIP occurs widely in the nervous system as well as in the gastrointestinal tract and exhibits a variety of biological activities, such as potent vasodilator activity, hypotensive activity, smooth muscle relaxing activity, intestinal juice secretion accelerating activity, pancreatic juice and bile secretion accelerating activity, gastric juice secretion inhibitory activity, glycogenolysis activity, various pituitary hormones secretion accelerating activity, blood flow regulating activity on the genital organs, bronchodilator activity, and so forth.

Of these various activities of VIP, smooth muscle relaxing activity on the bronchus and the trabeculae of the corpus cavernosum penis is attracting particular attention with the expectation of application to the treatment of bronchial asthma or impotence.

Production of VIP either by extraction and purification from animal organs or through chemical synthesis is accompanied by problems of stable supply, production efficiency, purity, production cost, and the like. Further, VIP is physicochemically labile and also tends to be degraded by enzymes present in the living body. It has therefore been considered difficult to put VIP alone to practical use as a drug.

In order to overcome these problems, active study has been directed to development of VIP analogues having biological activities equal to or higher than VIP or having higher stability than VIP and processes for chemically synthesizing such analogues.

The chemical structures of VIP analogues which have been reported thus far and the relationship of the structures and their biological activities are summarized below.

A polypeptide having the same structure as VIP except that methionine at the 17-position of VIP is displaced by norleucine possesses biological activities similar to those of VIP (see JP-A-62-246595, the term "JP-A" as used herein means an "unexamined published Japanese patent application").

Omission of one or two of the N-terminal amino acids and C-terminal amino acids from VIP results in considerable reduction in biological activities (see J. Biol. Chem., Vol. 266, pp. 6389-6392 (1991)), whereas addition of a basic amino acid to the C-terminal of VIP increases the biological activities by 3 to 4 times (see JP-A-62-246595).

A VIP analogue obtained by initially preparing a leading compound by acetylation of the N-terminal of VIP and displacement of methionine at the 17-position with norleucine and then displacing several amino acids of the leading compound with different amino acids exhibits excellent biological activities (see JP-A-3-38600).

However, since the above-described synthetic VIP analogues contain an amino acid(s) which does (do) not occur in nature, contain an additional amino acid or have an acetylated N-terminal, it is very likely that problems of production efficiency, product purity, and production cost, and the like may be posed. Further, there is the possibility that these synthetic VIP analogues, when employed in the human body, induce unfavorable side effects such as an antigen-antibody reaction. Furthermore, it is considered virtually impossible to prepare a VIP analogue containing a naturally-non-occurring amino acid or a VIP analogue with its N-terminal acetylated by genetic engineering.

In addition, there still is a demand for a further improvement in biological activities.

Genetic engineering is believed to be suited for production of VIP analogues with high degree of purity and at low cost on an industrial scale.

VIP has an amidated C-terminal and a helical structure on the whole, which seem to play an important role in manifestation of its biological activities (see Biochemistry, Vol. 28, pp. 2399-2409 (1989)). Thus, it would be safe to consider these structural features in the preparation of VIP or an analogue thereof by genetic engineering.

In general, the synthesis of a polypeptide or protein with an amidated C-terminal by genetic engineering involves amidation with an amidation enzyme, etc. in the final stage. Such an amidation treatment results in not only an increase in production cost but also a reduction in purity of the final product.

The inventors of the present invention have previously found that a VIP analogue with homoserine at the C-terminal thereof in non-amidated form exhibits biological activities equal to or superior to those of VIP (see EP-A-0 463 450A1). Radically differing from the conventional notion that C-terminal amidation is essential for VIP or an analogue thereof to manifest biological activities, the above finding is of great significance as to basic knowledge on preparing a VIP analogue by genetic engineering without an amidation step being involved.

The present inventors have also found that various polypeptides including motilin undergo a structural change due to a $\beta$-transfer reaction at the site of asparagine (Asn) or aspartic acid (Asp) during storage under usual conditions for drugs and that the structural change causes a reduction in purity. The finding implies the possibility of obtaining a VIP analogue with improved stability by replacing asparagine (Asn) or aspartic acid (Asp) at the 3-, 8-, 9- and 24-positions of naturally occurring VIP with other appropriate amino acids.

Naturally occurring VIP is very labile when stored under conditions usually employed for drugs and is easily degraded in vivo by proteolytic enzymes, etc. It should be administered at a high dose level because of its tendency toward degradation in vivo, and still the duration is short.

Synthetically prepared known VIP analogues, which seem to have overcome these disadvantages to some extent, are still unsatisfactory in terms of production cost and purity. Moreover, there is a need of further improvement in biological activities.

## SUMMARY OF THE INVENTION

An object of the present invention is to provide a polypeptide which is more stable than VIP, exhibits dozens of times a more potent biological activity as natural VIP, and is so designed as to be suitable for mass production by genetic engineering.

Another object of the present invention is to provide a drug, particularly a bronchodilator or a treating agent for impotence, comprising a polypeptide having VIP-like activities as an active ingredient with high efficiency and at a low cost.

As a result of extensive investigations, it has been found that replacement of aspartic acid (Asp) at the 8-position and asparagine (Asn) at the 24-position of natural VIP with glycine (Gly) and lysine (Lys) or glutamine (Gln), respectively, brings about an increase in stability while retaining high biological activities. It has also been found that appropriate arrangement of a basic amino acid, e.g., lysine (Lys) or arginine (Arg), at the 24-position or following positions and substitution of the C-terminal with homoserine provides a VIP analogue having biological activities increased by dozens of times over those of natural VIP.

Since VIP analogues according to the present invention contain no amino acids which do not occur in nature, and no chemical modification, such as acetylation of the N-terminal, they can be produced in large quantities with ease either by chemical synthesis method or genetic engineering techniques.

The present invention provides a polypeptide represented by formula (I) (SEQ. ID. NO. 1):

$$\text{His-Ser-Asp-Ala-Val-Phe-Thr-R}^1\text{-R}^2\text{-Tyr-Thr-R}^3\text{-Leu-Arg-}$$

$$\text{Lys-Gln-Leu-Ala-R}^4\text{-R}^5\text{-Lys-Tyr-Leu-R}^6\text{-R}^7\text{-R}^8\text{-R}^9\text{-R}^{10}\text{-X} \quad (\text{I})$$

wherein $R^1$ represents Asp or Gly; $R^2$ represents Asn or Gln; $R^3$ represents Arg or Lys; $R^4$ represents Val, Ala or Lys; $R^5$ represents Lys or Leu; $R^6$ represents Asn, Gln or Lys; $R^7$ represents Ser, Lys or Ala; $R^8$ represents Ile, Ala or Leu; $R^9$ represents Leu or Lys; $R^{10}$ represents Asn, Lys or Arg; and X represents homoserine inclusive of homoserine lactone (hereinafter abbreviated as Hse).

The polypeptides represented by formula (I) have a relaxing action on bronchial smooth muscles.

Among them, those represented by formula (I) wherein $R^1$ represents Gly; $R^2$ represents Asn; $R^3$ represents Lys; $R^4$ represents Ala; $R^5$ represents Lys; $R^6$ represents Lys or Gln; $R^7$ represents Lys; $R^8$ represents Ala; $R^9$ represents Leu; $R^{10}$ represents Lys or Arg; and X represents Hse exhibit particularly high bronchial smooth muscle relaxing activity.

3

## DETAILED DESCRIPTION OF THE INVENTION

The present invention will now be illustrated in greater detail with reference to the following Preparation Example and Test Examples.

## PREPARATION EXAMPLE

Various polypeptides according to the present invention were synthesized by starting with N-Boc-O-benzyl-L-homoseryl-4(oxymethyl)phenylacetic acid, which provides homoserine (Hse) at the C-terminal of a peptide after peptide synthesis followed by removal of a protective group and removal of a resin as an insoluble carrier in a conventional manner. The peptide synthesis was carried out using a peptide synthesizer "Model 431A" manufactured by Applied Biosystem Co. and a commercially available aminomethylated polystyrene•HCℓ resin as an insoluble carrier.

The protective group was removed from the resulting peptide in accordance with a trifluoromethanesulfonic acid method as described, for examples, in H. Yajima et al., Chem. Pharm. Bull., vol. 23, pp. 371-373-(1975), and the peptide was separated from the resin. The resulting peptide was purified by high performance liquid chromatography (HPLC) under the following conditions.

Column: "µ BONDASPHERE C-18" column (19 mm x 15 cm) manufactured by Waters Co.

Eluent: 0.012N HCℓ containing acetonitrile at a linear gradient concentration from 15% to 50%.

Flow rate: 7.0 mℓ/min x 35 min

The main peak fractions of HPLC were collected and lyophilized. The resulting peptide sample had homoserine or homoserine lactone at the C-terminal.

The purified polypeptides were automatically sequenced with a peptide sequencer (model 470A manufactured by Applied Biosystem Co.) and were confirmed the correct synthesis including the C-terminal homoserine.

The polypeptides of the present invention, which have homoserine (including homoserine lactone) at the C-terminal thereof, can be also produced in accordance with an ordinary genetic engineering technique.

The thus synthesized VIP analogues are shown in Table 1 below.

The VIP analogue of the present invention can be administered to humans or animals by inhalation, injection, percutaneous application, application to mucous membranes (e.g., the nasal cavity, eyes, the rectum, etc.) or oral administration. The VIP analogue can be formulated into various dose forms suitable for the route of administration using commonly employed methods of preparation. Typical but non-limiting dose forms include injectable solutions, suppositories, inhalations, capsules, tablets, powders, and eye drops.

## TEST EXAMPLE 1

### Bronchoconstriction Inhibitory Activity

The inhibitory activity of various VIP analogues obtained in the Preparation Example above on histamine-induced bronchoconstriction was evaluated in accordance with a Konzett-Rossler method (see Morooka S. et al., Br. J. Pharmacol., vol. 105, pp. 756-762 (1991)) as described below. VIP purchased from Peptide Kenkyusho K.K. was used as a reference substance. For comparison, a peptide with a non-acetylated N-terminal was synthesized based on the amino acid sequence disclosed in JP-A-3-38600 and evaluated in the same manner.

A test compound was directly infused into the cannulated respiratory tract of a guinea pig under urethane anesthesia, and the animal was set up for 1 minute so that the compound might reach the lungs. After 1.5 minutes from the administration, succinylcholine chloride was intravenously administered to suppress spontaneous respiration. Thirty minutes later, histamine (0.5 $\mu$g/kg) was intravenously injected. The 50% inhibitory dose ($ID_{50}$) of the test compound (a dose level with which histamine-induced constriction was 50% inhibited) was obtained according to a Konzett-Rossler method. The results obtained are shown in Table 1 below.

As is apparent from the results in Table 1, the VIP analogues having homoserine at the C-terminal thereof according to the present invention exhibit at least several times more potent bronchodilating activity as natural VIP. In particular, the polypeptides represented by

```
                                                    His-Ser-Asp-
Ala-Val-Phe-Thr-Gly-Asn-Tyr-Thr-Lys-Leu-Arg-Lys-Gln-Leu-Ala-
Ala-Lys-Lys-Tyr-Leu-R⁶-Lys-Ala-Leu-R¹⁰-X (SEQ. ID. NO. 2),
```

wherein R⁶ represents Lys or Gln; R¹⁰ represents Lys or Arg; and X represents Hse had a hundred times or more potent bronchodilating activity as natural VIP.

The bronchodilator activity of the comparative peptide with its non-acetylated N-terminal which was synthesized based on the amino acid sequence disclosed in JP-A-3-38600 was about one-third to eleventh that of the peptides represented by the above formula.

TEST EXAMPLE 2

Stability

Each of the VIP analogues as used in Test Example 1 was sterilely dissolved in a phosphate buffer to a final concentration varying from 0.1 to 1.0 mg/mℓ and sealed in a vial. The vial was stored in a thermostat set at 60°C for 10 days, and the change in the contents with time was evaluated by HPLC. The results obtained, as expressed in terms of residual activity with respect to the corresponding intact VIP or VIP analogue, are shown in Table 1 below.

It can be seen from the results in Table 1 that the peptides with Gly in place of the Asp at the 8-position and Lys or Gln in place of the Asn at the 24-position (a $\beta$-transfer reaction tends to occur at Asp or Asn at these and other positions) showed markedly improved stability over natural VIP.

TABLE 1

**Polypeptides**

Comparison

Natural type polypeptide *1:
His-Ser-Asp-Ala-Val-Phe-Thr-Asp-Asn-Tyr-Thr-Arg-Leu-Arg-Lys-Gln-Met-Ala-Val-Lys-Lys-Tyr-Leu-Asn-Ser-Ile-Leu-Asn-NH2 — In Vivo Activity ID50 (µg/kg) 7.5 — Residual Activity (%) 0-5

| | R1 | R2 | R3 | R4 | R5 | R6 | R7 | R8 | R9 | R10 | In Vivo Activity ID50 (µg/kg) | Residual Activity (%) |
|---|----|----|----|----|----|----|----|----|----|-----|---|---|
| Comparison | | | | | Leu | | | | | Hse | 2.5 | 0-5 |
| Comparison | | | Lys | Ala | Leu | Ala | Leu | Lys | Lys | - | 0.45 | 10-20 |
| Invention | | | Lys | Ala | Leu | Ala | Leu | Lys | Lys | Hse | 0.15 | 10-20 |
| Invention | Gly | | Lys | Lys | Leu | Ala | Leu | Lys | Lys | Hse | 0.75 | 40-50 |
| Invention | Gly | | Lys | Ala | Leu | | Lys | Ala | Lys | Hse*2 | 0.04 | 40-50 |
| Invention | Gly | | Lys | Ala | Leu | Gln | Lys | Ala | Lys | Hse | 0.07 | 50-60 |
| Invention | Gly | | Lys | Ala | Leu | | Lys | Ala | Lys | Hse | 0.04 | 10-20 |
| Invention | Gly | Gln | Lys | Ala | Leu | | Lys | Ala | Lys | Hse | 0.25 | 50-60 |
| Invention | Gly | Gln | Lys | Ala | Leu | Gln | Lys | Ala | Lys | Hse | 0.50 | 60-70 |
| Invention | Gly | | | Ala | Leu | | Lys | Ala | Lys | Hse | 0.08 | 40-50 |
| Invention | Gly | | Lys | Val | Leu | | Lys | Ala | Lys | Hse | 0.15 | 40-50 |
| Invention | Gly | | Lys | Ala | Leu | | Lys | Ala | Arg | Hse*3 | 0.03 | 40-50 |
| Invention | Gly | | Lys | Ala | Leu | | Lys | | | Hse | 0.09 | 40-50 |
| Invention | Gly | | Lys | Ala | Leu | Lys | Lys | Ala | Lys | Hse | 0.04 | 60-70 |
| Invention | Gly | | Lys | Ala | Leu | Lys | Lys | | Lys | Hse | 0.15 | 60-70 |
| Invention | Gly | | Lys | Ala | Leu | Lys | Lys | Ala | Arg | Hse | 0.04 | 60-70 |
| Invention | Gly | | Lys | Ala | Leu | Gln | Lys | Ala | Arg | Hse | 0.07 | 60-70 |
| Invention | Gly | | Lys | Ala | Leu | Lys | Lys | Ala | Arg | Hse | 0.04 | 30-40 |
| Invention | Gly | Gln | Lys | Ala | Leu | Lys | Lys | Ala | Arg | Hse | 0.15 | 70-80 |
| Invention | Gly | Gln | Lys | Ala | Leu | Gln | Lys | Ala | Arg | Hse | 0.40 | 70-80 |
| Invention | Gly | | Lys | Ala | Leu | Lys | Lys | Ala | Arg | Hse | 0.08 | 60-70 |
| Invention | Gly | | Lys | Val | Leu | Lys | Lys | Ala | Arg | Hse | 0.25 | 60-70 |

Note:  The amino acid sequence where not specified is the same as in natural VIP.
*1: Natural type polypeptide
*2: SEQ. ID. NO. 3
*3: SEQ. ID. NO. 4

The polypeptides according to the present invention exhibit at least several tens of times more potent biological activities such as bronchodilator activity than natural VIP. In addition, the polypeptides of the present invention have much longer duration as compared with conventional VIP and have greatly improved stability.

6

The polypeptides of the present invention, which have homoserine (including homoserine lactone) at the C-terminal thereof and possess VIP-like activities, can be produced at a low cost on an industrial scale by genetic engineering using E. coli, etc. as a host bacterium, taking advantage of the techniques previously developed by the inventors.

While the invention has been described in detail and with reference to specific examples thereof, it will be apparent to one skilled in the art that various changes and modifications can be made therein without departing from the spirit and scope thereof.

**Claims**

1.  A polypeptide represented by formula (I):

$$\text{His-Ser-Asp-Ala-Val-Phe-Thr-}R^1\text{-}R^2\text{-Tyr-Thr-}R^3\text{-Leu-Arg-}$$

$$\text{Lys-Gln-Leu-Ala-}R^4\text{-}R^5\text{-Lys-Tyr-Leu-}R^6\text{-}R^7\text{-}R^8\text{-}R^9\text{-}R^{10}\text{-X} \quad (I)$$

wherein $R^1$ represents Asp or Gly; $R^2$ represents Asn or Gln; $R^3$ represents Arg or Lys; $R^4$ represents Val, Ala or Lys; $R^5$ represents Lys or Leu; $R^6$ represents Asn, Gln or Lys; $R^7$ represents Ser, Lys or Ala; $R^8$ represents Ile, Ala or Leu; $R^9$ represents Leu or Lys; $R^{10}$ represents Asn, Lys or Arg; and X represents homoserine or homoserine lactone.

2.  A polypeptide as claimed in Claim 1, wherein $R^1$ represents Gly; $R^2$ represents Asn; $R^3$ represents Lys; $R^4$ represents Ala; $R^5$ represents Lys; $R^6$ represents Lys or Gln; $R^7$ represents Lys; $R^8$ represents Ala; $R^9$ represents Leu; $R^{10}$ represents Lys or Arg; and X represents homoserine or homoserine lactone.

3. A polypeptide as claimed in Claim 1, wherein $R^1$ represents Gly; $R^2$ represents Asn; $R^3$ represents Lys; $R^4$ represents Ala; $R^5$ represents Lys; $R^6$ represents Asn $R^7$ represents Lys; $R^8$ represents Ala; $R^9$ represents Leu; $R^{10}$ represents Lys; and X represents homoserine or homoserine lactone.

4. A polypeptide as claimed in Claim 1, wherein $R^1$ represents Gly; $R^2$ represents Asn; $R^3$ represents Lys; $R^4$ represents Ala; $R^5$ represents Lys; $R^6$ represents Asn; $R^7$ represents Lys; $R^8$ represents Ala; $R^9$ represents Leu; $R^{10}$ represents Arg; and X represents homoserine or homoserine lactone.

5. A pharmaceutical composition containing a polypeptide as claimed in Claim 1 as an active ingredient.

6. Use of a polypeptide as claimed in Claim 1 for the manufacture of a medicament to prevent or treat smooth muscle disorders.

7. Use of a polypeptide as claimed in Claim 1 for the manufacture of a medicament to treat or prevent bronchial asthma.

SEQUENCE LISTING

(1) GENERAL INFORMATION:

    (i) APPLICANT:
        (A) NAME: SANWA KAGAKU KENKYUSHO CO., LTD.
        (B) STREET: 35, Higashisotobori-cho, Higashi-ku
        (C) CITY: Nagoya-shi
        (D) STATE: Aichi
        (E) COUNTRY: Japan
        (F) POSTAL CODE (ZIP): 461
        (G) TELEPHONE: 052-951-8130
        (H) TELEFAX: 052-953-1241

    (ii) TITLE OF INVENTION: POLYPEPTIDE

   (iii) NUMBER OF SEQUENCES: 4

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)

    (vi) PRIOR APPLICATION DATA:
        (A) APPLICATION NUMBER: JP Hei. 5-009468
        (B) FILING DATE: 22-JAN-1993


(2) INFORMATION FOR SEQ ID NO: 1:

    (i) SEQUENCE CHARACTERISTICS:
        (A) LENGTH: 29 amino acids
        (B) TYPE: amino acid
        (D) TOPOLOGY: linear

    (ii) MOLECULE TYPE: peptide

    (xi) SEQUENCE DESCRIPTION: SEQ ID NO: 1:

His Ser Asp Ala Val Phe Thr Xaa Xaa Tyr Thr Xaa Leu Arg Lys Gln
1               5                   10                  15

Leu Ala Xaa Xaa Lys Tyr Leu Xaa Xaa Xaa Xaa Xaa Xaa
            20              25

(2) INFORMATION FOR SEQ ID NO: 2:

    (i) SEQUENCE CHARACTERISTICS:
        (A) LENGTH: 29 amino acids
        (B) TYPE: amino acid
        (D) TOPOLOGY: linear

    (ii) MOLECULE TYPE: peptide

    (xi) SEQUENCE DESCRIPTION: SEQ ID NO: 2:

```
His Ser Asp Ala Val Phe Thr Gly Asn Tyr Thr Lys Leu Arg Lys Gln
 1               5                 10              15

Leu Ala Ala Lys Lys Tyr Leu Xaa Lys Ala Leu Xaa Xaa
             20              25
```

(2) INFORMATION FOR SEQ ID NO: 3:

    (i) SEQUENCE CHARACTERISTICS:
       (A) LENGTH: 29 amino acids
       (B) TYPE: amino acid
       (D) TOPOLOGY: linear

    (ii) MOLECULE TYPE: peptide

    (xi) SEQUENCE DESCRIPTION: SEQ ID NO: 3:

```
His Ser Asp Ala Val Phe Thr Gly Asn Tyr Thr Lys Leu Arg Lys Gln
 1               5                 10              15

Leu Ala Ala Lys Lys Tyr Leu Asn Lys Ala Leu Lys Xaa
             20              25
```

(2) INFORMATION FOR SEQ ID NO: 4:

    (i) SEQUENCE CHARACTERISTICS:
       (A) LENGTH: 29 amino acids
       (B) TYPE: amino acid
       (D) TOPOLOGY: linear

    (ii) MOLECULE TYPE: peptide

    (xi) SEQUENCE DESCRIPTION: SEQ ID NO: 4:

```
His Ser Asp Ala Val Phe Thr Gly Asn Tyr Thr Lys Leu Arg Lys Gln
 1               5                 10              15

Leu Ala Ala Lys Lys Tyr Leu Asn Lys Ala Leu Arg Xaa
             20              25
```